# EUROPEAN PATENT APPLICATION

(11) **EP 2 518 047 A1**
(43) Date of publication of application: **31.10.2012**
(21) Application number: 10839532.8
(22) Date of filing: 24.12.2010
(51) Int. Cl.: C07C 213/02, C07C 41/22, C07C 41/30, C07C 43/188, C07C 43/192, C07C 215/44

(54) **METHOD FOR PRODUCING N-ALKYL- -VALIENAMINE ANALOGUE**

(30) Priority: 24.12.2009 JP 2009293008
(71) Applicant: SEIKAGAKU CORPORATION, Chiyoda-ku Tokyo 100-0005 (JP)
(72) Inventor: OTA, Naoki, Higashivamato-shi Tokyo 207-0021 (JP)
(74) Representative: HOFFMANN EITLE
(86) International application number: PCT/JP2010/073267
(87) International publication number: WO 2011/078302

(57) **Abstract**

There are provided a method for producing N-alkyl-β-valienamine analogs represented by formula (I): 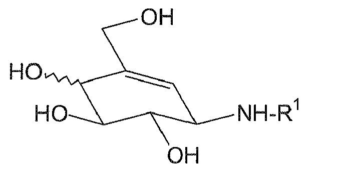 (where R¹ is a C₁₋₁₂ alkyl group), and useful novel intermediates for the production method.

## Description

### TECHNICAL FIELD

The present invention relates to a novel production method for N-alkyl-β-valienamine analogs, and to useful novel intermediates for the production method.

### BACKGROUND ART

Glycolipid metabolic disorders occur when normally metabolized intermediate metabolites accumulate due to loss or dysfunction of activity of various hydro lases in the lysosome. For example, GM1 gangliosidosis, Morquio B disease and Krabbe disease are known to occur due to loss or dysfunction of β-galactosidase (galactocerebrosidase) activity; Fabry disease is known to occur due to loss or dysfunction of α-galactosidase activity; Gaucher disease is known to occur due to loss or dysfunction of β-glucocerebrosidase (β-glucosidase) activity; and Tay-Sachs disease and Sandhoff disease (also known as GM2 gangliosidosis) are known to occur due to loss or dysfunction of β-hexosaminidase activity. Chemical chaperone therapy has been studied in recent years as a method of treatment for such glycolipid metabolic disorders.

Fabry disease patients, who suffer accumulation of sphingoglycolipids due to loss or dysfunction of α-galactosidase activity, have a mutant α-galactosidase enzyme, and the mutant enzyme is unstable and fails to exhibit sufficient activity. However, it has been reported that administering an α-galactosidase inhibitor at a lower concentration than that required for ordinary enzyme inhibition can stabilize the mutant enzyme and increase enzyme activity, and that an α-galactosidase inhibitor can thereby serve as an effective therapeutic agent for Fabry disease (see Non-Patent Literature 1, for example).

Similarly, carba-sugar amine derivatives, which are β-galactosidase and β-glucocerebrosidase inhibitors, are reported to have potential as effective therapeutic agents for glycolipid metabolic disorders resulting from loss or dysfunction of their enzyme activities (for example, GM1 gangliosidosis, Morquio B disease, Krabbe disease and Gaucher disease) (see Patent Literatures 1 and 2, for example).

Various methods have been reported to date for synthesis of such carba-sugar amines, and especially synthesis of N-alkyl-β-valienamine analogs such as N-octyl-β-valienamine (hereunder also referred to as "NOV") and N-octyl-4-epi-β-valienamine (hereunder also referred to as "NOEV") (see Patent Literatures 1 and 2, and Non-Patent Literatures 2 and 3).

[Patent Literature 1] International Patent Publication No. WO2004/1014
[Patent Literature 2] International Patent Publication No. WO2003/022797

[Non-Patent Literature 1] Jian-Qiang Fan et al., Nature Medicine, Vol.5, No.1, pages 112-115 (1999)
[Non-Patent Literature 2] Seiichiro Ogawa et al., Bioorganic & Medicinal Chemistry, Vol.12, No.5, pages 995-1002 (2004)
[Non-Patent Literature 3] Seiichiro Ogawa et al., Bioorganic & Medicinal Chemistry Letters, Vol.6, No.8, pages 929-932 (1996)

### SUMMARY OF INVENTION

### Technical Problem

The present invention provides a novel and industrially advantageous production method for N-alkyl-β-valienamine analogs, and novel intermediates that are useful for the production method.

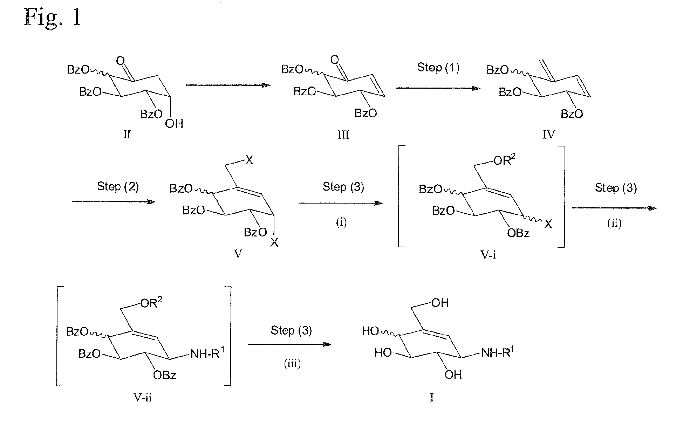

The hitherto reported methods for producing N-alkyl-β-valienamine analogs involve numerous steps and complex procedures, and therefore are not suitable for large-scale synthesis. For example, the Patent Literature 1 discloses a synthesis method for N-alkyl-β-valienamine analogs using methyl-α-D-glucopyranoside as the starting material, but as shown in Fig. 1 of the Patent Literature 1, the number of steps is large (for example, 26 steps are necessary to obtain NOEV), and the procedure is complicated, requiring column purification for all of the intermediates, and it is therefore difficult to scale-up the method for use as an industrial production method. A synthesis method for N-alkyl-β-valienamine analogs from different starting materials than that of the Patent Literature 1 is disclosed in the Patent Literature 2. However, in addition to the fact that the starting materials themselves are not readily available, the procedure is complicated, requiring column purification of all of the intermediates as in the Patent Literature 1, and therefore this method is also poorly suited for large-scale synthesis. The Non-Patented Literatures 2 and 3 disclose synthesis methods for NOEV and NOV, respectively, but similar to the methods mentioned above, the starting materials are difficult to obtain and the procedures are complicated, making them poorly suited for large-scale synthesis.

### Solution to Problem

We, the present inventors, have conducted diligent research in light of the aforementioned drawbacks of the prior art methods, with the aim of finding an industrially advantageous novel production method to reduce the number of steps and simplify the procedure in a method for producing for N-alkyl-β-valienamine analogs, and as a result we have found a novel method for producing N-alkyl-β-valienamine analogs via specific intermediates, and have completed this invention.

Specifically, the present invention relates to a method for producing N-alkyl-β-valienamine analogs represented by formula (I): 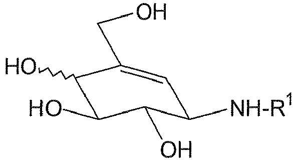 (where R¹ represents a C₁₋₁₂ alkyl group),
the method comprising the following steps (1) to (3):
(1) a step of homologation of a compound represented by formula (III): 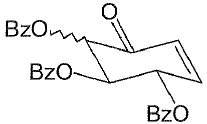 to obtain a compound represented by formula (IV): 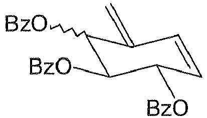
(2) a step of reacting a compound represented by formula (IV) with a halogenating reagent to obtain a compound represented by formula (V): 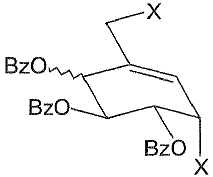 (where X represents a halogen atom), and
(3) a step of reacting a compound represented by formula (V) with (i) an organic acid salt, (ii) an alkylamine represented by the formula R¹NH₂ wherein R¹ is a C₁₋₁₂ alkyl group, and (iii) a strongly basic alkali metal salt in that order, to obtain a compound represented by formula (I).

The invention further relates to the aforementioned method wherein the homologation of step (1) is reaction with Nysted reagent.

The invention still further relates to the aforementioned method wherein the halogenating reagent of step (2) is pyridinium tribromide.

The invention still further relates to the aforementioned method wherein the (i) organic acid salt of step (3) is represented by the formula: R²CO₂M¹, where R² represents a C₁₋₆ alkyl, C₃₋₆ cycloalkyl, substituted or unsubstituted C₆₋₁₀ aryl or substituted or unsubstituted heteroaryl group, and M¹ represents an alkali metal or ammonium.

The invention still further relates to the aforementioned method wherein the (iii) strongly basic alkali metal salt of step (3) is an alkali metal alkoxide represented by the formula: M²-OR³, where R³ represents a C₁₋₆ alkyl group, and M² represents an alkali metal.

The invention still further relates to a compound represented by formula (IV).

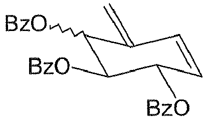

The invention still further relates to a compound represented by formula (V): 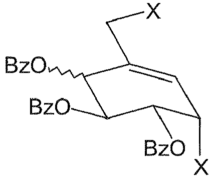 (where X is bromine).

The invention still further relates to a method for producing a compound represented by formula (V): 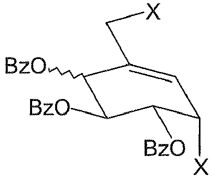 (where X represents a halogen atom),
wherein a compound represented by formula (III): 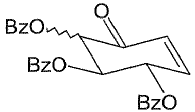 is reacted with Nysted reagent to obtain a compound represented by formula (IV): 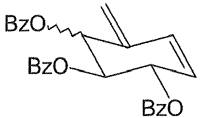 which is then reacted with a halogenating reagent.

The invention still further relates to a method for producing N-alkyl-β-valienamine analogs represented by formula (I): 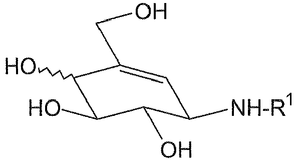 (where R¹ represents a C₁₋₁₂ alkyl group),
wherein a compound represented by formula (V): 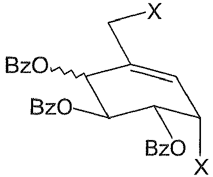 (where X represents a halogen atom)
is reacted with (i) an organic acid salt, (ii) an alkylamine represented by the formula: R¹NH₂, where R¹ represents a C₁₋₁₂ alkyl group, and (iii) a strongly basic alkali metal salt, in that order.

### Advantageous Effects of Invention

According to the production method of the invention, it is possible to obtain desired N-alkyl-β-valienamine analogs at high yield and high purity by only the simple procedures of partial purification and crystallization, without requiring complex and expensive procedures for industrial production, such as column chromatography purification. The production method of the invention is also superior because it allows safe, stable and inexpensive reagents to be selected. Thus, the method of the invention is suitable for large-scale synthesis and is promising as an industrially advantageous method.

### BRIEF DESCRIPTION OF DRAWINGS

Fig. 1 shows a scheme for a novel production method for N-alkyl-β-valienamine analogs according to the invention.

### DESCRIPTION OF EMBODIMENTS

The meanings of the terms used throughout the present specification and the claims will now be explained. The terms have the following meanings unless otherwise specified.

According to the invention, "halogen" refers to fluorine, chlorine, bromine or iodine, and preferably chlorine or bromine.

For R¹ according to the invention, "C₁₋₁₂ alkyl group" refers to a monovalent linear or branched saturated hydrocarbon group having 1-12 carbon atoms. Examples include methyl, ethyl, n-propyl, i-propyl, n-butyl, i-butyl, sec-butyl, tert-butyl or pentyl, hexyl, octyl, nonyl, decyl, undecyl, dodecyl, and their isomers. C1-8 alkyl groups such as methyl, ethyl, n-propyl, i-propyl, n-butyl, i-butyl, sec-butyl, tert-butyl, n-pentyl, n-hexyl and n-octyl are preferred, with linear C₁₋₈ alkyl groups being particularly preferred.

According to the invention, "C₁₋₆ alkyl group" means a monovalent linear or branched saturated hydrocarbon group having 1-6 carbon atoms, either alone or in combination with another group. Examples include methyl, ethyl, n-propyl, i-propyl, n-butyl, i-butyl, sec-butyl, tert-butyl or pentyl, hexyl, and their isomers. C₁₋₄ alkyl groups such as methyl, ethyl, n-propyl, i-propyl, n-butyl, i-butyl, sec-butyl and tert-butyl are preferred.

According to the invention, "C₃₋₆ cycloalkyl group" refers to a monovalent cyclic saturated hydrocarbon group having 3-6 carbon atoms. Examples include cyclopropyl, cyclobutyl, cyclopentyl and cyclohexyl.

According to the invention, "C₆₋₁₀aryl group" refers to a monovalent aromatic hydrocarbon group having 6-10 carbon atoms. Examples of such aryl groups include phenyl, indenyl and naphthyl. Phenyl or naphthyl is preferred.

According to the invention, "heteroaryl group" means a monovalent group of unsaturated monocyclic heterocycle having a 5- or 6-membered ring containing 1, 2 or 3 heteroatoms selected from among nitrogen, oxygen and sulfur, or a monovalent group of bicyclic heterocycle wherein the aforementioned monocyclic heterocycle is fused with benzene. Examples of such heteroaryl groups include monocyclic heterocyclic groups such as pyrrolyl, imidazolyl, pyrazolyl, furyl, thienyl, thiazolyl, oxazolyl, pyridyl, pyrazinyl, pyrimidinyl and pyridazinyl, and bicyclic heterocyclic groups such as indolyl, benzofuranyl, quinolyl and isoquinolyl.

The term "substituted" for the "aryl group" or "heteroaryl group" means that the "aryl group" or "heteroaryl group" is substituted with any desired substituent, such as 1-3 substituents selected from among halogen atoms, cyano, nitro, hydroxy, C₁₋₆ alkyl and C₁₋₆ alkoxy groups (where the alkyl portion is optionally substituted with a halogen atom).

A "C₁₋₆ alkoxy group" for the substituent is the group -O-R where R is a C₁₋₆ alkyl group. Examples of C₁₋₆ alkoxy groups include methoxy, ethoxy, n-propoxy, i-propoxy, n-butyloxy, i-butyloxy, sec-butyloxy, tert-butyloxy, pentyloxy and hexyloxy. C₁₋₄ alkoxy groups such as methoxy, ethoxy, n-propoxy, i-propoxy, n-butyloxy, i-butyloxy, sec-butyloxy and tert-butyloxy are preferred.

The term "alkyl portion is optionally substituted with a halogen atom" for the aforementioned substituents means that there are included modes wherein at least 1, and preferably 1-3, of the hydrogens on the hydrocarbon portion of the C₁₋₆ alkyl and C₁₋₆ alkoxy groups are substituted with identical or different halogen atoms. Examples of the former include fluoromethyl, difluoromethyl, trifluoromethyl, chloromethyl, dichloromethyl, trichloromethyl, 2-fluoroethyl and 2,2,2-trifluoroethyl. Examples of the latter include fluoromethoxy, difluoromethoxy, trifluoromethoxy, chloromethoxy, dichloromethoxy, trichloromethoxy, 2-fluoroethoxy and 2,2,2-trifluoroethoxy.

According to the invention, "alkali metal" means a typical element classified in Group 1A of the Periodic Table, including Li, Na and K. Na or K is preferred.

Embodiments of the invention will now be described.

### 1. N-alkyl-β-valienamine analogs

The invention relates to a method for producing N-alkyl-β-valienamine analogs represented by formula (I):

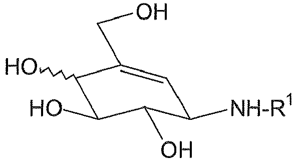

(where R¹ represents a C₁₋₁₂ alkyl group). Valienamine is a known carba-sugar amine represented by the following formula:

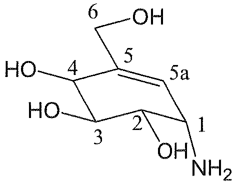

The N-alkyl-β-valienamine analogs of the invention include β-Glc-type compounds wherein the amino group at position 1 of valienamine is alkylated, and β-Gal-type compounds which are epimers at the hydroxyl group at position 4.

### 2. Starting substance

The production method of the invention starts from a compound represented by formula (III):

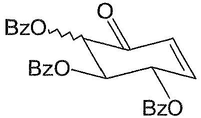

According to the invention, the abbreviation "Bz" in the chemical structural formulas represents a benzoyl group. The starting substance represented by formula (III) according to the invention can be obtained according to a known method. For example, according to R. Blattner et al., Carbohydrate Research, Vol. 150, Issue 1, pages 151-162 (1986*),* the compound represented by formula (IIa):

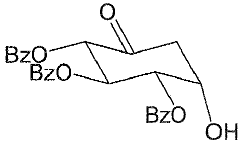

2L-(2,4,5/3)-2,3,4-tribenzoyloxy-5-hydroxycyclohexanone, may be treated with acetic anhydride in pyridine to obtain one compound represented by formula (III) according to the invention, represented by formula (IIIa).

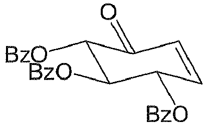

Similarly, the other compound represented by formula (III) of the invention, represented by formula (IIIb):

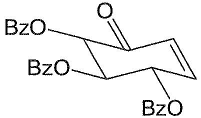

can be obtained from the compound represented by formula (IIb):

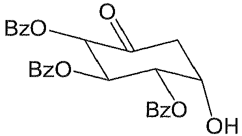

2D-(2,3/4,5)-2,3,4-tribenzoyloxy-5-hydroxycyclohexanone [which can be prepared by the method described in A. S. Machado et al., Carbohydrate Research, Vol. 135, Issue 2, pages 231-239 (1985*)].* Thus, the production method of the invention may employ a compound represented by formula (II):

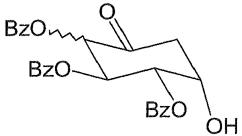

as the starting substance. In this case, the step of converting the starting substance represented by formula (II) to a compound represented by formula (III) by the method described above or another known method, such as the method described in Example 1 of the present specification, is also included in the production method of the invention.

### 3. Step (1): Formula (III) → formula (IV)

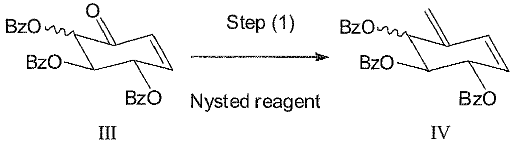

Step (1) is a step of converting the carbonyl group of a compound represented by formula (III) to an olefin. This conversion can be accomplished by methods commonly known to those skilled in the technical field of organic chemistry, by known homologation using a known olefinating reagent such as Wittig reagent, Horner-Emmons reagent, Tebbe reagent or Nysted reagent. In the production method of the invention, however, Nysted reagent, i.e. cyclo-dibromodi-µ-methylene[µ-(tetrahydrofuran)]trizinc, is used from the viewpoint of safety, stability and cost of the reagents and convenience of the treatment procedure. Nysted reagent is commercially available from reagent suppliers such as Sigma-Aldrich Corporation.

The reaction is carried out, for example, by treating a compound represented by formula (III) with Nysted reagent in an appropriate solvent selected from among ethers such as tetrahydrofuran (THF), dioxane, diethyl ether or dimethoxyethane, halogenated hydrocarbons such as methylene chloride, dichloroethane, chloroform or carbon tetrachloride, aromatic hydrocarbons such as benzene, toluene, xylene or monochlorobenzene, and mixtures of the foregoing solvents. Nysted reagent is used in a range of 1-5 equivalents and preferably 1-2 equivalents with respect to the compound represented by formula (III). There is no particular restriction on the reaction condition, and it may be conducted at a temperature of from -80°C to the boiling point of the solvent used, and preferably -20°C to 30°C for a period of from 0.1 hour to 7 days and preferably 0.1 to 5 hours.

The reaction is also conducted in the presence of titanium tetrachloride. The titanium tetrachloride is used in the range of 1-10 equivalents and preferably 1-4 equivalents with respect to the compound represented by formula (III).

Upon the completion of the reaction, the product is subjected to a common post-treatment procedure to obtain a crude product of a diene compound represented by formula (IV). The crude product may also be subjected to common purification such as column chromatography. However, it is preferably used as such in the subsequent step (2) without subjecting to special purification of the crude product. That is, steps (1) and (2) are preferably carried out consecutively.

### 4. Step (2): Formula (IV) → formula (V)

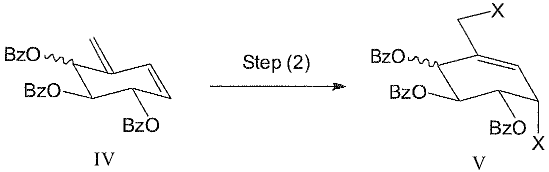

Step (2) is a 1,4-dihalogenation step for a diene compound represented by formula (IV). The dihalogenation may be carried out by a common method known to those skilled in the technical field of organic chemistry, using a known halogenating reagent. Such halogenating reagents include chlorine, bromine, iodine, sulfuryl chloride, pyridinium tribromide, dioxane dibromide and tetramethylammonium tribromide. From the viewpoint of safety and cost of the reagents and convenience of the treatment procedure, and from the viewpoint of reactivity of the obtained intermediate, a brominating reagent, and particularly pyridinium tribromide, is preferred.

The reaction is conducted by treating a compound represented by formula (IV) with a halogenating reagent in an appropriate solvent selected from among, for example, ethers such as THF, dioxane, diethyl ether and dimethoxyethane, halogenated hydrocarbons such as methylene chloride, dichloroethane, chloroform and carbon tetrachloride, esters such as methyl acetate, ethyl acetate and butyl acetate, aromatic hydrocarbons such as benzene, toluene, xylene and monochlorobenzene, and mixtures of the foregoing solvents. The halogenating reagent is used in the range of 1-10 equivalents and preferably 1-1.5 equivalents with respect to the compound represented by formula (IV) (or when steps (1) and (2) are carried out consecutively, this may be with respect to the compound represented by formula (III)). There is no particular restriction on the reaction condition, and it may be conducted at a temperature of from -80°C to the boiling point of the solvent used, and preferably 0°C to the boiling point of the solvent used, for a period of from 1 hour to 7 days and preferably 1 to 48 hours.

Upon the completion of the reaction, the product is subjected to a common post-treatment procedure to obtain a crude product of a dihalogeno compound represented by formula (V). The compound represented by formula (V) can be easily isolated and purified by crystallization, since it is a crystalline compound. Therefore, by conducting steps (1) and (2) in a consecutive manner and isolating and purifying the obtained compound represented by formula (V) by crystallization, it is possible to increase the purity of the final product of the production method of the invention. Compounds represented by formulas (IV) and (V) have UV absorption properties due to their benzoyl groups, and this may be utilized for quantitative process control. The properties of the compounds represented by formulas (IV) and (V) play an important role in providing an industrially advantageous novel production method for N-alkyl-β-valienamine analogs according to the invention.

### 5. Step (3)-(i): Formula (V) → formula (V-i)

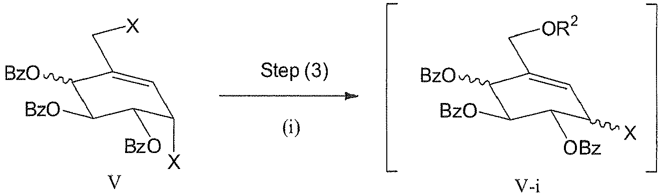

Step (3)-(i) is a monoacylation step for a dihalogeno compound represented by formula (V). The monoacylation can be conducted by a common method known to those skilled in the technical field of organic chemistry, using an appropriate organic acid salt. Such an organic acid salt is represented by the formula: R²CO₂M¹, where R² represents a C₁₋₆ alkyl, C₃₋₆ cycloalkyl, substituted or unsubstituted C₆₋₁₀ aryl or substituted or unsubstituted heteroaryl group, and M¹ represents an alkali metal or ammonium. Examples include sodium, potassium and ammonium salts of acetic acid, propionic acid, benzoic acid, toluic acid, xylylic acid, salicylic acid, anisic acid, nitrobenzoic acid and nicotinic acid. Preferred are organic acid salts represented by the formula R²CO₂M¹ where R² is a C₁₋₆ alkyl or substituted or unsubstituted C₆₋₁₀ aryl group. Examples of such organic acid salts include sodium acetate, potassium acetate, ammonium acetate, sodium benzoate, ammonium benzoate, sodium toluate and ammonium salicylate. These are commercially available from reagent suppliers such as Sigma-Aldrich Corp.

The reaction is conducted by treating a compound represented by formula (V) with an organic acid salt in an appropriate solvent selected from among, for example, alcohols such as methanol and ethanol, ketones such as acetone and methyl ethyl ketone (MEK), esters such as methyl acetate, ethyl acetate and butyl acetate, amides such as dimethylformamide (DMF), N-methylpyrrolidone (NMP) and dimethylacetamide (DMAc), methylcellosolve, ethylcellosolve, dioxane, dimethyl sulfoxide (DMSO), acetonitrile, and mixtures of the foregoing solvents. The organic acid salt is used in a range of 1-10 equivalents and preferably 1-1.2 equivalents with respect to the compound represented by formula (V). There is no particular restriction on the reaction condition, and it may be conducted, for example, at a temperature of from -80°C to the boiling point of the solvent used, and preferably 0°C to 40°C, and more preferably at ambient temperature, for a period of from 1 hour to 7 days and preferably 1 to 48 hours.

A tetraalkylammonium halide such as tetrabutylammonium iodide or tetrabutylammonium bromide may also be added as a catalyst. However, preferably a compound represented by formula (V) and an organic acid salt are reacted in the absence of a catalyst, using an amides such as dimethylacetamide (DMAc).

In the reaction of step (3)-(i), a small amount of the β-form monoacyl compound (by-product) represented by formula (V-i) is formed together with the α-form monoacyl compound represented by formula (V-i) (main product) (confirmed by high-performance liquid chromatography (HPLC)). Upon the completion of the reaction, a common post-treatment procedure may be carried out to obtain a crude product of the monoacyl compound represented by formula (V-i). The crude product may also be subjected to a common purification procedure such as column chromatography for separation into the α-form or β-form of formula (V-i). However, the presence of the by-product has no particular effect on the subsequent reaction steps, and after confirming the completion of the reaction of step (3)-(i) by common analysis such as thin-layer chromatography (TLC), liquid chromatography (LC) and HPLC, the reaction mixture is preferably used as such in the subsequent step (3)-(ii). That is, upon the completion of the reaction of step (3)-(i), the reaction mixture is preferably used for the reaction of step (3)-(ii) in a consecutive manner.

### 6. Step (3)-(ii): Formula (V-i) → formula (V-ii)

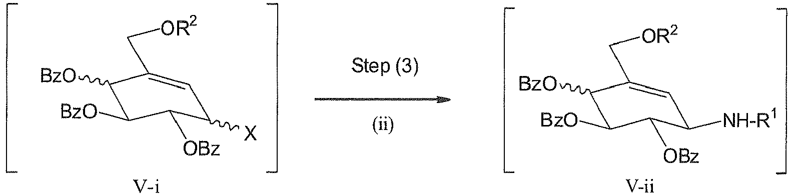

Step (3)-(ii) is a step of alkylamination of the monoacyl-monohalogeno compound represented by formula (V-i). The alkylamination may be carried out by a common method known to those skilled in the technical field of organic chemistry, using an appropriate alkylamine represented by the formula: R¹NH₂ where R¹ represents a C₁₋₁₂ alkyl group. Examples of such alkylamines include methylamine, ethylamine, n-propylamine, i-propylamine, n-butylamine, i-butylamine, sec-butylamine, tert-butylamine, n-pentylamine, n-hexylamine, n-octylamine and n-dodecylamine. These are commercially available from reagent suppliers such as Sigma-Aldrich Corp.

The reaction is conducted by treating a compound represented by formula (V-i) with an alkylamine in an appropriate solvent selected from among, for example, alcohols such as methanol and ethanol, ketones such as acetone and MEK, esters such as methyl acetate, ethyl acetate and butyl acetate, amides such as DMF, NMP and DMAc, methylcellosolve, ethylcellosolve, dioxane, DMSO, acetonitrile, and mixtures of the foregoing solvents. According to a preferred embodiment, the reaction can be carried out using as such the reaction mixture obtained upon the completion of the reaction of step (3)-(i). That is, it is preferably carried out in the same solvent as step (3)-(i). The alkylamine is used in the range of 1-20 equivalents and preferably 2-10 equivalents with respect to the compound represented by formula (V-i) (or when steps (3)-(i) and (ii) are carried out consecutively, this may be based on the compound represented by formula (V)). There is no particular restriction on the reaction condition, and it may be conducted at a temperature of from 0°C to the boiling point of the solvent used, and preferably 10°C to 50°C, for a period of from 1 hour to 7 days and preferably 1 to 24 hours.

Upon the completion of the reaction, a common post-treatment procedure may be carried out to obtain a crude product of the alkylamino compound represented by formula (V-ii). The crude product may also be subjected to common purification such as column chromatography. However, the reaction mixture as such is preferably used for the subsequent step (3)-(iii) after confirming the completion of the reaction of step (3)-(ii) by common analysis such as TLC or LC. That is, upon the completion of the reaction of step (3)-(i), the reaction mixture is preferably used for the reaction of steps (3)-(ii) and (iii) in a consecutive manner.

### 7. Step (3)-(iii): Formula (V-ii) → formula (I)

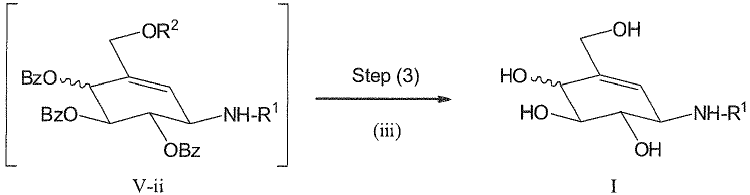

Step (3)-(iii) is a deprotection step for the compound represented by formula (V-ii). The deprotection can be conducted by a common method known to those skilled in the technical field of organic chemistry, using an appropriate strongly basic alkali metal salt. Such strongly basic alkali metal salts are preferably alkali metal alkoxides represented by the formula: M²-OR³, where R³ represents a C1-6 alkyl group, and M² represents an alkali metal, and examples include sodium methoxide, sodium ethoxide, potassium ethoxide and potassium tert-butoxide. These are commercially available from reagent suppliers such as Sigma-Aldrich Corp., or they may be prepared from alkali metals and alcohols at the time of use.

The reaction is conducted by treating a compound represented by formula (V-ii) with a strongly basic alkali metal salt in an appropriate solvent selected from among, for example, alcohols such as methanol and ethanol, ketones such as acetone and MEK, esters such as methyl acetate, ethyl acetate and butyl acetate, amides such as DMF, NMP and DMAc, methylcellosolve, ethylcellosolve, dioxane, DMSO, acetonitrile, and mixtures of the foregoing solvents. According to a preferred embodiment, the reaction can be carried out using as such the reaction mixture obtained upon the completion of the reaction of step (3)-(ii). That is, it is preferably carried out in the same solvent as step (3)-(ii). The strongly basic alkali metal salt is used in the range of 1-20 equivalents and preferably 1-10 equivalents with respect to the compound represented by formula (V-ii) (or when steps (3)-(i) to (iii) are carried out consecutively, this may be based on the compound represented by formula (V)). There is no particular restriction on the reaction condition, and it may be conducted at a temperature of from 0°C to the boiling point of the solvent used, and preferably 10°C to 50°C, for a period of from 1 hour to 7 days and preferably 1 to 24 hours.

Upon the completion of the reaction, a common post-treatment procedure may be carried out to obtain a crude product of an N-alkyl-β-valienamine analog represented by formula (I). The crude product may also be subjected to common purification such as column chromatography.

### EXAMPLES

The present invention will now be explained in further detail by examples, with the understanding that the scope of the invention is not limited to the examples.
Identification of the compounds was accomplished by ¹H-NMR, HPLC and LC/MS. The measuring conditions were as follows.

### [HPLC]

· Column: Reversed-phase, Mightysil RP-18 250-4.6 (5 µm), product of Kanto Kagaku Co., Ltd.
· Mobile phase: Acetonitrile:water = 85:15
· Flow rate: 0.5 ml/min
· Detection wavelength: 230 nm
· Temperature: 40°C.
· Analysis time: 25 min

### [LC/MS]

· Column: Reversed-phase, XBridge^{™} C18, 5 µm, 2.1 × 50 mm (product of Waters Co.)
· Mobile phase: Methanol (A):20 mmol/L ammonium acetate water (B)
· Flow rate: 0.5 ml/min (with a constant speed gradient in the following 3 stages)
· First stage: (A)/(B) = 50/50-95/5 (time: 1 min)
· Second stage: (A)/(B) = 95/5 (time: 3.5 min, constant speed, constant ratio)
· Third stage: (A)/(B) = 50/50 (time: 0.5 min)
· Temperature: 40°C.
· Analysis time: 5 min
· Ionization method: ESI⁺
· Cone voltage: +1 V (without limit for voltage without fragmentation)
·m/z measurement range: 100-2000

### [Example 1]

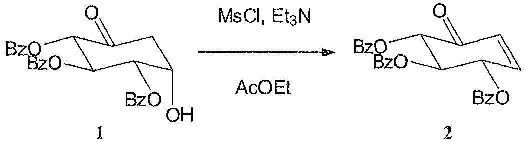

After adding 23.68 g (49.91 mmol) of compound 1 and 360 ml of ethyl acetate in a 1 L three-necked flask, the mixture was heated and stirred to dissolution at a bath temperature of approximately 50°C. After confirming dissolution, the mixture was cooled on ice until the internal temperature reached approximately 10°C, and then 25.25 g (249.5 mmol, 5.0 eq) of triethylamine (Et₃N) was added while stirring. After the internal temperature reached approximately 7°C, 11.43 g (99.82 mmol, 2.0 eq) of methanesulfonyl chloride (MsCl) was added dropwise over a period of about 6 minutes while stirring (the internal temperature increased to a maximum of 20°C). The ice bath was removed, the mixture was stirred for about 20 minutes at an internal temperature of approximately 20°C to 22°C, and the completion of the reaction was confirmed by TLC (developing solvent: hexane/ethyl acetate = 1/1, developing conditions: heating while dipped in sulfuric acid/ethanol= 1/9, Rf= 0.7 (compounds 1), 0.9 (compound 2)).
The reaction mixture (ethyl acetate layer) was extracted, separated and rinsed with 118 ml of water, 118 ml of 1N aqueous hydrochloric acid (removing the excess Et₃N and confirming hydrochloric acidity with pH test paper) and 118 ml of water, in that order. After rinsing, the organic layer was dried over magnesium sulfate (rinsing with 50 ml of ethyl acetate). The solvent was removed at a bath temperature of 40°C and 40 mmHg, to obtain a concentrated residue of compound 2 (yellowish-brown oil, partially crystallized). After adding 118 ml of methanol to the residue (with crystal precipitation immediately following addition), the mixture was stirred at approximately 22°C for 1 hour and at approximately 5°C for 1 hour. The crystals were filtered out and rinsed with 47 ml of cold methanol. This was dried under reduced pressure at about 22°C to obtain 18.80 g (82.5%) of compound 2 as faint yellow crystals.

### · Melting point

151-152°C

### ·¹H-NMR

δ(CDCl₃(500 MHz)) 7.26-8.02 (m, 15H, Bz x 3), 7.03 (dd, 1H, J_{4,3}=10.5 Hz, J_{4,6}=2.0 Hz, H-4), 6.37 (dd, 1H, J_{3,2}=2.5 Hz, J_{3.4}=10.5 Hz, H-3), 6.33 (ddd, 1H, J_{2,1}=9.0 Hz, J_{2,3}=2.5 Hz, J_{2,6}=2.0 Hz, H-2), 6.25 (dd, 1H, J_{1,2}=9.0 Hz, J_{1,6}=11.5 Hz, H-1), 5.96 (d, 1H, J_{6,1}=11.5 Hz, H-6)

### ·LC/MS

492 (M+NH₄⁺), 966 (2 M+NH₄⁺)

### [Example 2]

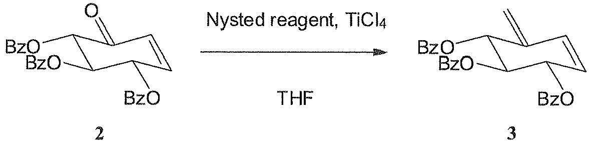

After adding 17.00 g of Nysted reagent (20 wt% THF suspension, product of Aldrich, 7.45 mmol) to a 100 ml round-bottomed, short-necked flask, it was stirred while cooling on ice. Separately, 2.00 g (4.38 mmol) of compound 2 was dissolved in 10 ml of dehydrated THF, and the solution was added to the flask containing Nysted reagent. After the internal temperature reached approximately 1°C, 8.8 ml of titanium tetrachloride (ca. 1.0 M dichloromethane solution, ca. 8.8 mmol) was added dropwise over a period of about 5 minutes while stirring (the internal temperature increased to a maximum of 21°C). The ice bath was removed, stirring was continued for about 1.5 hours at an internal temperature of approximately 25°C to 29°C, and the completion of the reaction was confirmed by HPLC.
The reaction mixture was cooled on ice, and after the internal temperature reached approximately 7°C, 5 ml of methanol was added dropwise over a period of about 5 minutes (the internal temperature increased to a maximum of 16°C). The reaction mixture was returned to room temperature (approximately 22°C), and the solvent was distilled off at a bath temperature of 40°C and 40 mmHg. To the concentrated residue (black tar) there were added 20 ml of ethyl acetate, 10 ml of water, 15 ml of 3N aqueous hydrochloric acid and 10 ml of 10 wt% aqueous solution of sodium chloride, and the mixture was stirred, allowed to stand and separated. After separation, the organic layer was dried over magnesium sulfate (rinsing with 10 ml of ethyl acetate). The solvent was removed at a bath temperature of 40°C and 40 mmHg, to obtain a crude product of compound 3 (reddish brown oil). The crude product was purified by TLC (20 cm × 20 cm PLC plate by Merck, Silica Gel 60 F254, 2 mm, developing solvent: hexane/ethyl acetate = 3/1) to obtain 1.65 g (82.9%) of compound 3 as faint yellow oil.

### ¹H-NMR

δ(CDCl₃(500 MHz)) 7.25-8.01 (m, 15H, Bz x 3), 6.43 (dd, 1H, J_{4,3}=10.5 Hz, J_{4,6a}=2.0 Hz, H-4), 6.19 (dd, 1H, J_{3,2}=2.0 Hz, J_{3,4}=10.5 Hz, H-3), 6.07 (dd, 1H, J_{2,1}=8.0 Hz, J_{2,3}=2.0 Hz, H-2), 5.94 (dd, 1H, J_{1,2}=8.0 Hz, J_{1,5a}= 10. Hz, H-1), 5.89 (d, 1H, J_{5a, 1}=10.5 Hz, H-5a), 5.29 (d, 1H, J_{6a,4}=2.0 Hz, H-6a), 5.22 (s, 1H, H-6b)

### ·LC/MS

474 (M+NH₄⁺), 926 (2 M+NH₄⁺)

### [Example 3]

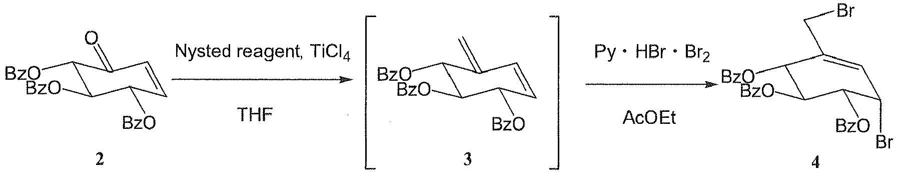

After adding 313.26 g of Nysted reagent (20 wt% THF suspension, product of Aldrich, 136.93 mmol) to a 1 L three-necked flask, it was stirred while cooling on ice-salt. Separately, 50.00 g (109.54 mmol) of compound 2 was dissolved in 150 ml of dehydrated THF, and the solution was added to the flask containing Nysted reagent. After the internal temperature reached approximately -7°C, 137 ml of titanium tetrachloride (ca. 1.0 M dichloromethane solution, ca. 137 mmol) was added dropwise over a period of about 45 minutes while stirring (the internal temperature increased to a maximum of 4°C). The ice-salt was removed, the mixture was stirred for about 30 minutes at an internal temperature of approximately 4°C to 22°C, and the completion of the reaction was confirmed by HPLC (producing compound 3). The reaction mixture was cooled on ice, and after the internal temperature reached approximately 6°C, 100 ml of methanol was added dropwise over a period of about 15 minutes (the internal temperature increased to a maximum of 19°C). The reaction mixture was returned to room temperature (approximately 22°C), and the solvent was distilled off at a bath temperature of 40°C and 40 mmHg. After adding 200 ml of ethyl acetate to the concentrated residue (black tar), the mixture was cooled on ice. After the internal temperature reached approximately 9°C, 150 ml of 3N aqueous hydrochloric acid was added dropwise over a period of about 10 minutes (the internal temperature increased to 17°C). The reaction mixture was returned to room temperature (approximately 22°C), and then 200 ml of toluene was added and the mixture was stirred, allowed to stand and separated. After separation, the organic layer was stored, 100 ml of toluene was again added to the hydrochloric layer, and the mixture was stirred, allowed to stand and separated, after which all of the organic layers were combined. After rinsing the organic layer with 20 wt% aqueous solution of sodium chloride, it was dried over magnesium sulfate (rinsing with 100 ml of ethyl acetate). The solvent was removed at a bath temperature of 40°C and 40 mmHg, to obtain 58.72 g of a crude product of compound 3 as reddish brown oil. This was used for the subsequent reaction without further purification.

After adding 58.72 g of the crude compound 3 and 250 ml of ethyl acetate to a 1 L three-necked flask, the mixture was stirred and dissolved. After adding 42.04 g (131.45 mmol) of pyridinium tribromide and stirring for about 18 hours at an internal temperature of approximately 75°C, the completion of the reaction was confirmed by HPLC (producing compound 4). The reaction mixture was returned to room temperature (approximately 22°C), 200 ml of 10 wt% aqueous sodium thiosulfate was added, and the mixture was stirred, allowed to stand and separated. After separation, the organic layer was stored, 100 ml of ethyl acetate was again added to the aqueous layer, and the mixture was stirred, allowed to stand and separated, after which all of the organic layers were combined. After adding sodium sulfate and 5.00 g of active carbon (Tokusei Shirasagi) to the organic layer, the mixture was stirred for about 10 minutes at a bath temperature of approximately 75°C. The insoluble portion was filtered out and rinsed with 100 ml of ethyl acetate, and the solvent was distilled off at a bath temperature of 40°C and 40 mmHg. After adding 250 ml of methanol to the concentrated residue, the mixture was stirred at a bath temperature of 70°C (gradual deposition of crystals). Stirring was continued at room temperature (approximately 22°C) for about 30 minutes and while cooling on ice (approximately 5°C) for about 2.5 hours, and then the crystals were filtered and rinsed with 100 ml of methanol. This was dried under reduced pressure at 40°C to obtain 32.34 g of compound 4 as faint brown crystals (2-step yield: 48.1 %).

### · Melting point

148-149°C

### ·¹H-NMR

δ(CDCl₃(500 MHz)) 7.26-7.98 (m, 15H, Bz x 3), 6.56 (d, 1H, J_{4,3}=8.0 Hz, H-4), 6.37 (d, 1H, J_{5a, 1}=5.5 Hz, H-5a), 6.26 (dd, 1H, J_{3,2}=10.5 Hz, J_{3,4}=8.0 Hz, H-3), 5.39 (dd, 1H, J_{2,1}=4.0 Hz, J_{2,3}=10.5 Hz, H-2), 5.21 (dd-like-t, 1H, J_{1,2}=4.0 Hz, J_{1,5a}=5.5 Hz, H-1), 4.09 (d, 1H, J_{gem}=11.0 Hz, H-6a), 3.97 (d, 1H, J_{gem}=11.0 Hz, H-6b)

### ·LC/MS

630, 632, 634 (M+NH₄⁺)

### [Example 4]

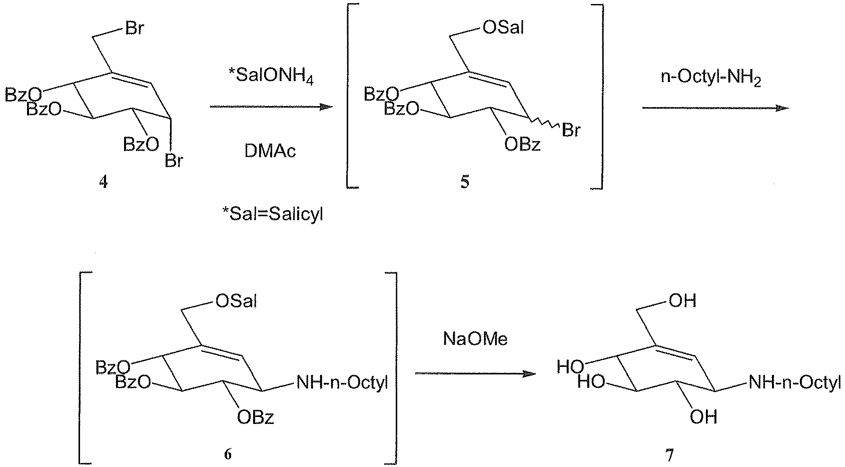

Compound 4 (30.00 g, 48.84 mmol) was dissolved in 60 ml of DMAc. After adding 8.71 g (56.14 mmol, 1.15 eq) of ammonium salicylate to the solution, the mixture was stirred at an internal temperature of approximately 20°C to 22°C for 17 hours. After confirming the completion of the reaction with HPLC (producing compound 5), 31.56 g (244.18 mmol, 5.0 eq) of n-octylamine was added and the mixture was stirred at an internal temperature of approximately 45°C for 4.5 hours. After confirming the completion of the reaction by LC/MS (producing a mixture of compound 6 and its sugar-deprotected form), 15.83 g (293.04 mmol, 6.0 eq) of sodium methoxide and 60 ml of methanol were added and the mixture was stirred at an internal temperature of approximately 45°C for 1 hour. After confirming the completion of the reaction by LC/MS (producing NOV), the methanol was distilled off at a bath temperature of 40°C and 30 mmHg.

To the distilled residue solution there were added 45 ml of methanol and 105 ml of water, and the n-octylamine was azeotropically distilled off with water for about 1 hour at a bath temperature of 40°C and 30 mmHg. The azeotropic procedure was repeated 5 times in total, and distillation of n-octylamine was confirmed by LC/MS. The residue of the azeotropic distillation was cooled on ice, and then 150 ml of 2N aqueous hydrochloric acid and 150 ml of ethyl acetate were added and the mixture was stirred, allowed to stand and separated. After separation, the hydrochloric aqueous layer was stored, 60 ml of 2N aqueous hydrochloric acid was again added to the ethyl acetate layer, the mixture was stirred, allowed to stand and separated, and all of the hydrochloric aqueous layers were combined.

To the hydrochloric aqueous layer there was slowly added 19.79 g (186.72 mmol) of sodium carbonate while stirring, and weak basicity was confirmed with pH test paper. After adding 40.51 g (693.19 mmol) of sodium chloride while stirring and confirming that the sodium chloride no longer dissolved, 150 ml of tetrahydrofuran was added and the mixture was stirred, allowed to stand and separated. After separation, the tetrahydrofuran layer was stored, 90 ml of tetrahydrofuran was again added to the aqueous layer, the mixture was stirred, allowed to stand and separated (the procedure being repeated 3 times in total), and all of the tetrahydrofuran layers were combined.

The tetrahydrofuran was distilled off at a bath temperature of 40°C and 30 mmHg, and the residue was dissolved in 90 ml of ethanol. After adding 5.00 g of sodium sulfate and 4.50 g of active carbon (Tokusei Shirasagi) to the solution, the mixture was stirred at a bath temperature of at 40°C for 5 minutes. The insoluble portion was distilled off and rinsed with 60 ml of ethanol. The ethanol was distilled off at a bath temperature of 40°C and 30 mmHg, and the residue was dissolved in 30 ml of ethanol. The solution was subjected to microfiltration (0.45 µm filter) and the ethanol was distilled off at a bath temperature of 40°C and 30 mmHg. After adding 90 ml of ethyl acetate to the residue and precipitating a gum-like solid, a suitable amount of dry ice was added and the solid was dispersed with a spatula. The internal temperature was returned to approximately 20°C, and the ethyl acetate was decanted. After again adding 90 ml of ethyl acetate and a suitable amount of dry ice, the solid was dispersed with a spatula. The internal temperature was returned to approximately 20°C, and the ethyl acetate was decanted. Following decantation, the residue was dried under reduced pressure overnight with a vacuum pump to obtain 4.73 g (33.7%) of compound 7 (NOV) as a yellowish white amorphous substance.

### ·¹H-NMR

δCDCl₃/MeOD=2/1 (500 MHz)) 5.61 (s, 1H, H-5a), 4.11 and 4.19 (d, 2H, J_{gem}=14.0 Hz, H-6a, b), 4.18 (d, 1H, J_{4,3}=7.5 Hz, H-4), 3.55 (dd, 1H, J_{3,2}=10.0 Hz, J_{3,4}=7.5 Hz, H-3), 3.43 (dd, 1H, J_{2,1}=8.5 Hz, J_{2,3}=10.0 Hz, H-2), 3.23 (d, 1H, J_{1,2}=8.5 Hz, H-1), 2.76 and 2.56 (ddd, 2H, NHCH₂(CH₂)₆CH₃), 1.28-1.54 (m, 12H, NHCH₂(CH2)₆CH₃), 0.89 (t, 3H, J=7.0 Hz, NHCH₂(CH₂)₆CH₃)

### ·LC/MS

288 (M+1), 576 (2M+1)

### INDUSTRIAL APPLICABILITY

According to the production method of the invention, it is possible to obtain N-alkyl-β-valienamine analogs at high yield and high purity by only the simple procedures of partial purification and crystallization, without requiring complex and expensive procedures for industrial production, such as column chromatography purification. The N-alkyl-β-valienamine analogs obtained by the production method of the invention are promising as therapeutic agents for glycolipid metabolic disorders. The method of the invention is suitable for large-scale synthesis and is expected to be an industrially advantageous synthetic method, and is promising for commercial industry as well.

## Claims

1. A method for producing N-alkyl-β-valienamine analogs represented by formula (I): 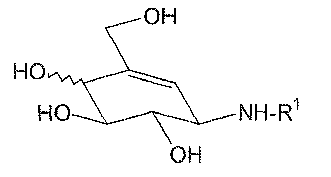 (wherein R¹ represents a C₁₋₁₂ alkyl group),
the method comprising the following steps (1) to (3):
(1) a step of homologation of a compound represented by formula (III): 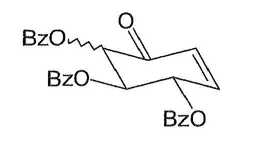 to obtain a compound represented by formula (IV): 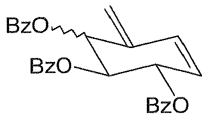
(2) a step of reacting a compound represented by formula (IV) with a halogenating reagent to obtain a compound represented by formula (V): 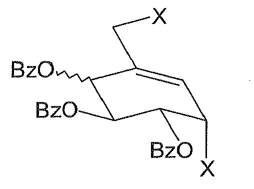 (wherein X represents a halogen atom), and
(3) a step of reacting a compound represented by formula (V) with (i) an organic acid salt, (ii) an alkylamine represented by the formula R¹NH₂ wherein R¹ is a C₁₋₁₂ alkyl group, and (iii) a strongly basic alkali metal salt in that order, to obtain a compound represented by formula (I).

2. The method according to claim 1, wherein the homologation of step (1) is reaction with Nysted reagent.

3. The method according to claim 1 or 2, wherein the halogenating reagent of step (2) is pyridinium tribromide.

4. The method according to any one of claims 1 to 3, wherein the (i) organic acid salt of step (3) is represented by the formula: R²CO₂M¹, where R² represents a C₁₋₆ alkyl, C₃₋₆ cycloalkyl, substituted or unsubstituted C₆₋₁₀ aryl or substituted or unsubstituted heteroaryl group, and M¹ represents an alkali metal or ammonium.

5. The method according to any one of claims 1 to 4, wherein the (iii) strongly basic alkali metal salt of step (3) is an alkali metal alkoxide represented by the formula: M²-OR³, where R³ represents a C₁₋₆ alkyl group, M² represents an alkali metal.

6. A compound represented by formula (IV).

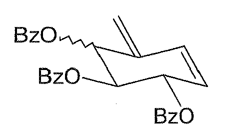

7. A compound represented by formula (V): 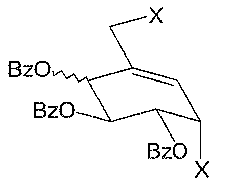 (wherein X is bromine).

8. A method for producing a compound represented by formula (V): 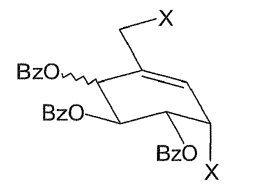 (where X represents a halogen atom),
wherein a compound represented by formula (III): 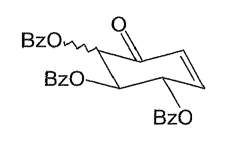 is reacted with Nysted reagent to obtain a compound represented by formula (IV): 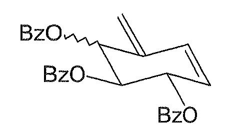 which is then reacted with a halogenating reagent.

9. A method for producing N-alkyl-β-valienamine analogs represented by formula (I): 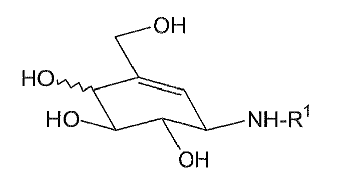 (where R¹ represents a C₁₋₁₂ alkyl group),
wherein a compound represented by formula (V): 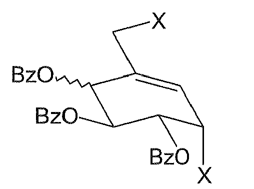 (where X represents a halogen atom)
is reacted with (i) an organic acid salt, (ii) an alkylamine represented by the formula: R¹NH₂, where R¹ represents a C₁₋₁₂ alkyl group, and (iii) a strongly basic alkali metal salt, in that order.
